(19) **Europäisches Patentamt / European Patent Office / Office européen des brevets**

(11) **EP 1 408 951 B1**

(12) **EUROPEAN PATENT SPECIFICATION**

(45) Date of publication and mention
of the grant of the patent:
**30.12.2009 Bulletin 2009/53**

(51) Int Cl.:
*A61K 47/08* (2006.01)     *A61K 39/39* (2006.01)
*A61K 47/44* (2006.01)     *A61K 31/20* (2006.01)

(21) Application number: **02761953.5**

(22) Date of filing: **11.04.2002**

(86) International application number:
**PCT/IL2002/000295**

(87) International publication number:
**WO 2002/083058 (24.10.2002 Gazette 2002/43)**

(54) **ANTI-INFLAMMATORY FATTY ALCOHOLS AND FATTY ACID ESTERS USEFUL AS ANTIGEN CARRIERS**

ENTZÜNDUNGSHEMMENDE FETTALKOHOLE UND FETTSÄUREESTER ALS ANTIGENTRÄGER

ALCOOLS GRAS ANTI-INFLAMMATOIRES ET ESTERS D'ACIDE GRAS UTILES EN TANT QUE PORTEURS D'ANTIGENES

(84) Designated Contracting States:
**AT BE CH CY DE DK ES FI FR GB GR IE IT LI LU MC NL PT SE TR**
Designated Extension States:
**AL LT LV MK RO SI**

(30) Priority: **11.04.2001  IL 14253601**

(43) Date of publication of application:
**21.04.2004  Bulletin 2004/17**

(73) Proprietor: **YEDA RESEARCH AND DEVELOPMENT CO., LTD.**
**76100 Rehovot (IL)**

(72) Inventors:
• **COHEN, Irun, R.**
**76354 Rehovot (IL)**

• **SHINITZKY, Meir**
**46910 Kfar Shmaryahu (IL)**
• **MARGALIT, Raanan**
**76922 Ganei Yochanan (IL)**

(74) Representative: **Vossius & Partner**
**Siebertstrasse 4**
**81675 München (DE)**

(56) References cited:
**EP-A- 1 385 500     WO-A-99/04632**
**US-A- 4 152 423     US-A- 5 910 306**
**US-A- 5 948 764     US-A- 6 114 337**

• **Heeger et al. (2000) J. Immunol. 164, 5771-5781.**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 408 951 B1

**Description**

**FIELD OF THE INVENTION**

The present invention relates to therapeutic preparations for treatment of T-cell mediated diseases or conditions and, in particular, to the use of an anti-inflammatory immunomodulator selected from a fatty alcohol, an ester thereof with a $C_1$-$C_6$ alkanoic acid, or an ester of a fatty acid with an alkanol or a polyol, as a carrier for antigens recognized by inflammatory T cells associated with said T-cell mediated diseases or conditions.

**ABBREVIATIONS: AA:** adjuvant arthritis: **CFA:** complete Freund's adjuvant; **EAE:** experimental autoimmune encephalomyelitis; **IFA:** incomplete Freund's adjuvant; **MBP:** myelin basic protein; **MOG:** myelin oligodendrocyte glycoprotein; **OA:** oleyl alcohol; **PLP:** proteolipid protein **SC:** subcutaneously; **TCR:** T-cell receptor.

**BACKGROUND OF THE INVENTION**

Lymphocytes are the central cells of the immune system, responsible for acquired immunity and the immunologic attributes of diversity, specificity, memory, and self/nonself recognition. Mature B cells are distinguished from other lymphocytes by their synthesis and display of membrane-bound immunoglobulin (antibody) molecules, which serve as receptors for antigens. Interaction between antigen and the membrane-bound antibody on a mature naive B cell, results in the activation and differentiation of B-cell clones of corresponding specificity and the consequent production of B cell clones lacking the membrane-bound antibody but which secrete antibody molecules with the same antigen-binding specificity.

T lymphocytes, like B lymphocytes, have membrane receptors for antigen. However, unlike the membrane-bound antibody on B cells, the T-cell receptor (TCR) does not recognize free antigen. Instead the TCR recognizes only antigen that is bound to a self-molecule encoded by genes within the major histocompatibility complex (MHC). To be recognized by most T cells, the antigen must be displayed together with MHC molecules on the surface of antigen-presenting cells (APC) or on virus-infected cells, cancer cells, and grafts.

Like B cells, T cells express distinctive membrane molecules. All T-cell subpopulations express the TCR, a complex of polypeptides that includes CD3, and most can be distinguished by the presence of one or the other of two membrane molecules, CD4 and CD8. T cells that express the membrane glycoprotein molecule CD4 are restricted to recognizing antigen bound to class II MHC molecules, whereas T cells expressing CD8, a dimeric membrane glycoprotein, are restricted to recognition of antigen bound to class I MHC molecules

In general, expression of CD4 and of CD8 also defines two major subpopulations of T lymphocytes. CD4$^+$ T cells generally function as T helper ($T_H$) cells and are class-II restricted; CD8$^+$ T cells generally function as T cytotoxic ($T_C$) cells and are class-I restricted;

$T_H$ cells are activated by recognition of an antigen-class II MHC complex on an antigen-presenting cell. After activation, the $T_H$ cell begins to divide and gives rise to a clone of effector cells, each specific for the same antigen-class II MHC complex. These $T_H$ cells secrete various cytokines, which play a central role in the activation of B cells, T cells, and other cells that participate in the immune response.

Changes in the pattern of cytokines produced by $T_H$ cells can change the type of immune response that develops among other leukocytes. Thus $T_H$ cells have been divided into two groups by the characteristic cytokines they secrete when activated (Mosmann and Coffman, 1989). The $T_H1$ response produces a cytokine profile that supports inflammation and activates mainly certain T cells and macrophages whereas the $T_H2$ response activates mainly B cells and immune responses that depend upon antibodies. Thus, $T_H1$ cells secrete IL-2, which induces T-cell proliferation, and cytokines such as IFN-γ, which mediates tissue inflammation. $T_H2$ cells, in contrast, secrete IL-4, which activates B cells to secrete antibodies of certain IgG isotypes and suppresses the production of $T_H1$ inflammatory cytokines (Banchereau et al., 1994), and IL-10, which suppresses inflammatory cytokine production by macrophages and thus indirectly reduces cytokines production by $T_H1$ cells and affects antigen-presenting cells by down-regulating class II MHC expression (Moore et al., 1993).

Autoimmunity results from an inappropriate response of the immune system against self-components leading to activation of self-reactive clones of T or B cells, and generation of humoral or cell-mediated responses against endogenous antigens, with consequent injury to cells, tissues and organs. Sometimes the damage is caused by antibodies as in the autoimmune disorders Addison's disease, autoimmune anemias e.g. autoimmune hemolytic anemia and pernicious anemia, Hashimoto's thyroiditis and scleroderma.

Many autoimmune disorders e.g. insulin-dependent diabetes mellitus (IDDM or type I diabetes), multiple sclerosis, rheumatoid arthritis and autoimmune thyroiditis are characterized by tissue destruction mediated by T cells activated by an endogenous antigen. These immune responses to self-antigens are maintained by the persistent activation of the self-reactive T lymphocytes.

Autoimmune diseases can be divided into organ-specific autoimmune diseases, in which the immune response is directed to a target antigen unique to a single organ or gland, so that the manifestations are largely limited to that organ, and systemic autoimmune diseases, in which the response is directed toward a broad range of target antigens and involves a number of organs and tissues. Examples of organ-specific autoimmune diseases include insulin-dependent diabetes mellitus, multiple sclerosis, rheumatoid arthritis, thyroiditis, and myasthenia gravis, and examples of systemic autoimmune diseases include systemic lupus erythematosus and scleroderma.

It is the $T_H1$ cells which contribute to the pathogenesis of organ-specific autoimmune diseases. For example, there is strong evidence that, in mice, experimental autoimmune encephalomyelitis (EAE) is caused by CD4$^+$ $T_H1$ cells specific for the immunizing antigen e.g. myelin basic protein (MBP) or proteolipid protein (PLP). The disease can be transferred from one animal into another by T cells from animals immunized with either MBP or PLP or by cloned T-cell lines from such animals. $T_H1$-type responses also appear to be involved in other T-cell mediated diseases or conditions such as contact dermatitis (Romagnani, 1994).

Most cases of organ-specific autoimmune diseases develop as a consequence of self-reactive CD4$^+$ T cells. Analysis of these T cells revealed that the $T_H1$/ $T_H2$ balance can affect whether autoimmunity develops. $T_H1$ cells have been involved in the development of autoimmunity, whereas, in several cases, $T_H2$ cells not only protected against the induction of the disease but also against progression of established disease and in the induction and maintenance of allograft tolerance.

Several therapeutic approaches have been explored for treatment of autoimmune diseases. Identification and sequencing of various autoantigens has led to the development of new approaches to modulate autoimmune T-cell activity. Whole antigens involved in the pathogenesis of the autoimmune disease or peptides derived from their sequence have been proposed for the treatment of autoimmune diseases.

Synthetic peptides suitable for immunologically specific therapy of an autoimmune disease are peptides that are recognized by T cells involved in the pathogenesis of the autoimmune disease. These peptides may have a sequence consisting of a pathogenic sequence within the sequence of an antigen involved in the disease or may be an analogue thereof, in which sequence one or more native amino acid residues are substituted by different amino acid residues, particularly a so-called "altered peptide", which contains a single amino acid substitution in the epitope of the pathogenic native counterpart (i.e., the region that contacts the TCR), but have no alterations in the agretope (i.e., the region that contacts the MHC).

Each autoimmune disease will have its ideal peptide for use in therapy that is derived directly from the sequence of an antigen associated with the disease or is an altered peptide or another analogue thereof. Thus, a disease like multiple sclerosis (MS) involving T cells reactive to self-antigens such as myelin basic protein (MBP), myelin oligodendrocyte glycoprotein (MOG) and proteolipid protein (PLP) will require for its therapy a peptide of MBP, MOG or PLP or an analogue thereof; myasthenia gravis can be treated with a peptide from the acetylcholine receptor; thyroiditis with a peptide from thyroglobulin; diabetes type 1 with a peptide of glutamic acid decarboxylase (GAD) or a peptide from the insulin sequence; systemic lupus erythematosus with a peptide derived from the protein P53; and Guillain-Barré syndrome with a peptide from the myelin antigen P2.

In recent years, peptides derived from a pathogenic self-antigen associated with an autoimmune disease or analogues thereof have been proposed for treatment of the disease. For example, peptides derived from the human MBP sequence (US 5,817,629, US 6,250,040) and analogues thereof (US 5,948,764; US 6,329,499) have been described for treatment of multiple sclerosis; peptide analogues of the 65 kD isoform of human GAD and of insulin have been proposed for treatment of diabetes (US 5,945,401 and US 6,197,926, respectively); and an autoantigen or a fragment thereof have been described for the treatment of uveoretinitis (US 5,961,977).

In each of the various autoimmune diseases, it would be desirable to administer the relevant peptide in an adjuvant that would activate T cells of the anti-inflammatory $T_H2$ phenotype. This would be expected to arrest the autoimmune process (see Liblau et al., 1995). There are also situations not involving therapy of an autoimmune disease in which it would be useful to activate specific T cells with a $T_H2$ phenotype. However, treatments involving self-antigens must be done in adjuvants that do not induce $T_H1$-type immunity that might activate dangerous $T_H1$ autoimmunity in the treated subject. Thus, there is a need to identify adjuvants capable of being combined with specific antigens that will induce non-inflammatory $T_H2$-type T cells.

Adjuvants, by their nature, are non-specific immunomodulators. An adjuvant suitable for the purposes outlined above would be a non-specific immunomodulator that could be combined in a therapeutic vaccination with an antigen or other molecule so as to induce the activation of specific T cells of the desired anti-inflammatory phenotype.

Several peptides suitable for the therapy of T-cell mediated diseases or conditions such as autoimmune diseases were shown to be effective when administered to mice subcutaneously (SC) in an oil vehicle such as an emulsion of mineral oil known as incomplete Freund's adjuvant (IFA) (Ben-Nun and Cohen, 1982). However, IFA as well as complete Freund's adjuvant (CFA; a preparation of mineral oil containing various amounts of killed organisms of Mycobacterium) are not allowed for human use because the mineral oil cannot be degraded in the body.

The present inventors have found previously that a metabolizable fat emulsion comprising 10-20% triglycerides of plant and/or animal origin, 1.2-2.4% phospholipids of plant and/or animal origin, 2.25-4.5% osmo-regulator, 0-0.05% antioxidant, and sterile water to complete 100 ml, such as the lipid emulsions known as Intralipid and Lipofundin, can be used as adjuvant in combination with specific antigens for the therapy of T-cell mediated diseases or conditions such as autoimmune diseases (WO 97/020016).

US 5,019,383 describe synthetic vaccines comprising a peptide residue coupled to one or more alkyl or alkenyl groups of at least 12 carbon atoms or other lipophilic substance, wherein said alkyl or alkenyl group may be a fatty acid residue coupled to one or more functional groups of a polyfunctional group which is bound to the N-terminal animo group and/or C-terminal carboxy group of the peptide residue.

US-A 5,984,764 discloses methods for treatment of multiple sclerosis utilizing peptide analogues of human myelin basic protein.

WO 99/04632 relates to immunization methods using the recombinant viral vectors, it discloses methods and compositions for immunizing a subject with a nucleic acid molecule encoding an antigen of interest, wherein the nucleic acid is delivered to the subject via a recombinant AAV virion.

Heeger et al (2000) J. Immunol. 164, 5771-5781 relates to tolerance induced by autoantigen in incomplete Freund's Adjuvant (IFA).

It would be highly desirable to discover effective vehicles for peptide therapy that would be degradable and act as anti-inflammatory immunomodulators.

## SUMMARY OF THE INVENTION

It has now been found, in accordance with the present invention, that curtain long-chain fatty alcohols, esters thereof with $C_1$ - $C_6$ alkanoic acids, or certain esters of long-chain fatty acids with alkanols and polyols act as anti-inflammatory immunomodulators and, therefore, can be used as adjuvants in combination with specific antigens to activate T cells for the purpose of therapy of autoimmune diseases and for T-cell mediated immune effects that need preferably a $T_H2$-type immune response.

The present invention thus relates, in one embodiment, to a therapeutic preparation comprising an antigen and a carrier, wherein the antigen is one recognized by inflammatory T cells associated with the pathogenesis of said T-cell mediated disease or condition, and wherein the carrier is an anti-inflammatory immunomodulator selected from: (a) a saturated or cis-unsaturated $C_{10}$-$C_{20}$ fatty alcohol or an ester thereof with a $C_1$-$C_6$ alkanoic acid; and (b) a saturated or cis-unsaturated $C_{10}$-$C_{20}$ fatty acid ester wherein said ester is selected from a $C_1$-$C_6$ alkyl ester, a monoester with a $C_2$-$C_6$ polyol having a least two hydroxy groups, and a diester with glycerol.

The antigen to be used together with the carrier of the invention is one recognized by inflammatory T cells associated with the pathogenesis of an autoimmune disease or other T-cell mediated disease or condition, and may be a peptide or an non-peptidic antigen.

In another embodiment, the invention relates to a therapeutic preparation as defined above which causes shifting of an individual's T-cell cytokine response from $T_H1$ to $T_H2$.

In as further embodiment, the invention relates to a therapeutic preparation as defined above which causes a decrease in IL-2 and/or IFN-$\gamma$ T-cell cytokine response and an increase in IL-4 and/or IL-10 T-cell cytokine response. In a further embodiment the invention relates to the therapeutic preparation as defined above for the treatment of a T-cell mediated disease or condition.

In still another embodiment, the invention prelates to the use of (i) a carrier which is an anti-inflammatory immunomodulator selected from: (a) a saturated or cis-unsaturated $C_{10}$-$C_{20}$ fatty alcohol or an ester thereof with a $C_1$-$C_6$ alkanoic acid; or (b) a saturated or cis-unsaturated $C_{10}$-$C_{20}$ fatty acid ester wherein said ester is selected from a $C_1$-$C_6$ alkyl ester, a monoester with a $C_2$-$C_6$ polyol having a least two hydroxy groups, or a diester with glycerol, and (ii) an antigen recognized by inflammatory T cells associated with the pathogenesis of a T-cell mediated disease or condition, for the manufacture of a therapeutic preparation for the treatment of said T-cell mediated disease or condition.

In yet still another embodiment, the invention relates to a method for the treatment of a patient suffering from a T-cell mediated disease or condition, which comprises administering to said patient a therapeutic preparation comprising an antigen recognized by inflammatory T cells associated with the pathogenesis of said T-cell mediated disease or condition and a biologically active carrier consisting of a lipid emulsion of an anti-inflammatory immunomodulator selected from: (a) a saturated or cis-unsaturated $C_{10}$-$C_{20}$ fatty alcohol or an ester thereof with a $C_1$-$C_6$ alkanoic acid; and (b) a saturated or cis-unsaturated $C_{10}$-$C_{20}$ fatty acid ester wherein said ester is selected from a $C_1$-$C_6$ alkyl ester, a monoester with a $C_2$-$C_6$ polyol having a least two hydroxy groups, and a diester with glycerol.

## BRIEF DESCRIPTION OF THE DRAWING

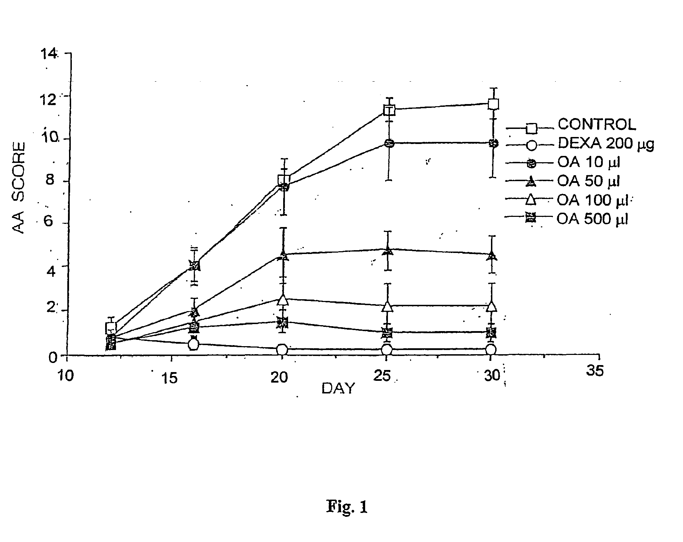

Fig. 1 shows the dose response effect of oleyl alcohol (OA) on adjuvant arthritis (AA). Different doses of OA were administered subcutaneously to rats once 14 days before induction of AA.

## DETAILED DESCRIPTION OF THE INVENTION

In the description and claims herein the terms "carrier" and "adjuvant" are used as synonyms meaning a non-specific immunomodulator that can be combined with an antigen in a therapeutic vaccine for induction of activation of specific T cells of the desired anti-inflammatory phenotype.

The present invention provides an anti-inflammatory immunomodulator useful as adjuvant together with an antigen recognized by inflammatory T cells associated with the pathogenesis of a T-cell mediated disease or condition, for the manufacture of a therapeutic preparation for the treatment of said T-cell mediated disease or condition.

The adjuvant for use according to the invention is an anti-inflammatory immunomodulator selected from: (a) a saturated or cis-unsaturated $C_{10}$-$C_{20}$ fatty alcohol or an ester thereof with a $C_1$-$C_6$ alkanoic acid; and (b) a saturated or cis-unsaturated $C_{10}$-$C_{20}$ fatty acid ester wherein said ester is selected from a $C_1$-$C_6$ alkyl ester, a monoester with a $C_2$-$C_6$ polyol having a least two hydroxy groups, and a diester with glycerol.

According to one preferred embodiment of the invention, the adjuvant is a long-chain saturated or unsaturated $C_{10}$-$C_{20}$, preferably $C_{16}$-$C_{20}$, most preferably a $C_{18}$, fatty alcohol. Examples of saturated fatty alcohols that can be used according to the invention include, but are not limited to, decyl alcohol, lauryl alcohol, myristyl alcohol, stearyl alcohol, and preferably cetyl alcohol (also known as palmityl alcohol). The unsaturated fatty alcohol has preferably one or more double bonds in the cis form and 16-18 carbon atoms such as, but not being limited to, oleyl alcohol (cis-9-octadecenol), linoleyl alcohol (cis-9,12-octadecadienol), γ-linolenyl alcohol (cis-6,9,12-octadecatrienol) and linolenyl alcohol (cis-9,12,15-octadecatrienol). In preferred embodiments, the fatty alcohol is cetyl, linolenyl or, most preferably, oleyl alcohol.

In another embodiment, the adjuvant of the invention is an ester of a fatty alcohol as defined above with a $C_1$-$C_6$ alkanoic acid such as acetic acid, propionic acid, butyric acid, valeric acid and caproic acid.

In a further embodiment, the adjuvant of the invention is an ester of a saturated or cis-unsaturated $C_{10}$-$C_{20}$ fatty acid, wherein said ester is selected from a $C_1$-$C_8$ alkyl ester, a monoester with a polyol having at least two hydroxy groups, and a diester with glycerol, wherein one or two of the three hydroxy groups of glycerol are esterified with said saturated or cis-unsaturated long-chain fatty acid.

The $C_{10}$-$C_{20}$ fatty acid is preferably a $C_{16}$-$C_{20}$, most preferably a $C_{18}$ fatty acid. In one embodiment, the $C_{10}$-$C_{20}$ fatty acid is saturated such as, but without being limited to, capric acid, lauric acid, myristic acid, palmitic acid, stearic acid and arachidic acid. In another embodiment, the $C_{10}$-$C_{20}$ fatty acid is a cis-unsaturated fatty acid such as, but without being limited to, palmitoleic acid (cis-9-hexadecenoic acid), oleic acid (cis-9-octadecenoic acid), cis-vaccenic acid (cis-11-octadecenoic acid), linoleic acid (cis-9,12-octadecadienoic acid), γ-linolenic acid (cis-6,9,12-octadecatrienoic acid), linolenic acid (cis-9,12,15-octadecatrienoic acid) and arachidonic acid (cis- 5,8,11,14-eicosatetraenoic acid). In one preferred embodiment, the fatty acid is oleic acid.

The alkyl ester of the saturated or cis-unsaturated $C_{10}$-$C_{20}$ fatty acid may have 1-6 carbon atoms in the alkyl chain and may be, without being limited to, methyl or ethyl. In preferred embodiments, the alkyl ester is methyl oleate or ethyl oleate.

In a further embodiment, the adjuvant of the invention is a monoester of a saturated or cis-unsaturated $C_{10}$-$C_{20}$ fatty acid with a polyol having at least two hydroxy groups, such as a $C_2$-$C_8$ alkanediol, glycerol or a saccharide.

The $C_2$-$C_8$ alkanediol has preferably 2 to 4, most preferably 2, carbon atoms and may be 1,2-ethylene glycol, 1,3-propanediol and 1,4-butanediol. An example of such an ester is 1,2-ethylene glycol monooleate.

The saccharide may be, for example, without being limited to, a monosaccharide such as ribose, fructose, glucose, galactose or mannose. An example of such an ester is mannose monooleate.

According to another embodiment of the invention, the adjuvant is a mono- or diester of glycerol with the $C_{10}$-$C_{20}$ fatty acid. In the case of monoglycerides, e.g. glyceryl monooleate, the resulting ester will contain two free hydroxyl groups. The diglycerides contain one free hydroxyl group and the other two hydroxyl groups may be both esterified with 2 molecules of the $C_{10}$-$C_{20}$ fatty acid, e.g. glyceryl dioleate, or one of the hydroxyl groups is esterified with one molecule of the $C_{10}$-$C_{20}$ fatty acid and a second hydroxyl group is esterified with one molecule of another carboxylic acid such as, but not being limited to, aliphatic $C_2$-$C_6$ carboxylic acids, e.g. acetic, propanoic, butyric and hexanoic acids.

The anti-inflammatory immunomodulators of the present invention form lipid emulsions that, when used as a vaccine adjuvant with the antigenic substance to which the T cells involved in the disease or condition being treated are active, serve to mediate a shift from a $T_H1$ T-cell response prior to treatment to a $T_H2$ T-cell response after treatment. This finding establishes that such lipid emulsions are tolerogenic biologically active carriers which can be used in vaccines for the treatment of any $T_H1$-mediated disease or condition. In such vaccines, the antigen provides the immunological specificity for a therapeutic effect while the biologically active carrier of the present invention provides the biological outcome, i.e. the $T_H1 \rightarrow T_H2$ shift. Because of the shift mediated by said biologically active carrier of the present invention, diseases with a spectrum of autoreactivities can be turned off with a single antigen/carrier combination capable of inducing a T-cell cytokine shift.

A preferred use in accordance with the present invention of the therapeutic preparation comprising an antigen and the anti-inflammatory immunomodulator adjuvant is in the treatment of organ-specific autoimmune diseases which are mediated by $T_H1$ cells. Such diseases include, but are not limited to, autoimmune diseases such as multiple sclerosis, diabetes type I, rheumatoid arthritis, and thyroiditis.

The antigen used in the preparation is an antigen recognized by inflammatory T cells associated with the pathogenesis of said autoimmune disease and may be the whole protein involved in the disease process, a peptide derived from the sequence of such a protein, an altered peptide which has a single amino acid substitution in the epitope of the pathogenic autoantigen peptide, or any other peptide recognized by the inflammatory T cells associated with the disease.

Thus, for the treatment of multiple sclerosis, the antigen could be MBP, MOG or PLP, or a peptide derived from the human MBP sequence such as the peptides MBP(75-95), MBP(86-95), and MBP(82-98) described in US 5,817,629, or analogues thereof described in US 5,948,764 and 6,239,499, or MOG peptides or PLP peptides and analogues thereof. The antigen could also be Copolymer I or Cop 1, a random copolymer composed of the four amino acids: tyrosine-glutamate-alanine-lysine, that cross-reacts functionally with MBP and is able to compete with MBP on the MHC class II in the antigen presentation. Cop 1, in the form of its acetate salt known under the generic name Glatiramer acetate, has been approved in several countries for the treatment of MS under the trade name, COPAXONE® (a trademark ofTeva Pharmaceuticals Ltd., Petah Tikva, Israel).

For the treatment of diabetes type I (IDDM), the peptide may be derived from glutamic acid decarboxylase (GAD), or GAD peptide analogues as described in US 5,945,401, or insulin peptide analogues such as the peptides comprising residues 9 to 23 of the native insulin B chain sequence which are altered at position 12, 13, 15 and/or 16 and may be further altered as described in US 6,197,926.

For the treatment of other autoimmune diseases the peptide will be derived from the sequence of an antigen associated with the disease or a peptide analogue thereof. Thus, for treatment of autoimmune thyroiditis, the peptide will be derived from the sequence of thyroglobulin; for rheumatoid arthritis the autoantigen can be derived from collagen II or from a Mycobacterium organism, e.g. *Mycobacterium tuberculosis,* e.g. the 60 kDa heat shock protein known as hsp60, which constitutes the mycobacterial epitope recognized by T lymphocytes in adjuvant arthritis, or the corresponding human HSP60 or a peptide thereof; for the treatment of myasthenia gravis, the peptide is derived from the sequence of the acetylcholine receptor or an analogue thereof as described in US 6,066,621; for the treatment of systemic lupus erythematosus the peptide may be derived from the sequence of the protein P53; and for the treatment of Guillain-Barré syndrome the peptide may be derived from the sequence of myelin antigen P2.

The antigen may be a non-peptidic antigen. Examples of non-peptidic antigens that can be used according to the invention include, but are not limited to, phospholipids for the treatment of phospholipid syndrome, cholesterol for the treatment of atherosclerosis, and DNA molecules for the treatment of systemic lupus erythematosus.

It is not critical that the antigen be a peptide. Thus, for example, $T_H1$ mediated allergic responses which result in skin sensitivity and inflammation, such as contact dermatitis, can be treated by a vaccine containing the irritant antigen and a biologically active carrier in accordance with the present invention which will cause a shift in the cytokine response from a $T_H1$-type to a $T_H2$-type. Thus, while the patient will continue to have elevated antibody levels against the antigen, the inflammatory T-cell response causing the skin irritation will be suppressed.

Accordingly, the tolerogenic biologically active carrier of the present invention may be used any time that it is desired to create tolerance for the antigen which the T cells are attacking, i.e., any time that a vaccine is being used to restrict a T-cell mediated condition, particularly a $T_H1$-cell mediated condition. If it can be determined which antigen is activating the response in graft rejection or in graft-versus-host disease, then the administration of such an antigen with a carrier in accordance with the present invention would be expected to facilitate the shift of the undesirable inflammatory $T_H1$ response to a more desirable $T_H2$ response, regardless of the overall complexity of the number of antigens to which T cells are active in such condition.

To determine the T-cell secretion of cytokines following activation with peptides, lymphocytes from the peripheral blood of patients are tested in an *in vitro* activation assay. Peripheral blood lymphocytes are isolated from whole heparinized blood on fico-hypaque, and cultured with the test peptide(s) at concentration of 5-50 $\mu$g/ml. The supernatants from the cultured T-cells are collected at different time points and tested for activity of various cytokines, by ELISA or bioassay(s).

The finding according to the present invention that long-chain fatty alcohols and esters thereof as well as certain fatty acid esters may be used effectively as adjuvants for T-cell activation, is completely unexpected. Similarly, the discovery that these preparations are tolerogenic biologically active anti-inflammatory immunomodulators is also totally unexpected.

The advantage of adding an antigen and so using an immunomodulator as described herein as adjuvant is in the fact that the treatment can be limited to the relatively short exposure required to induce a protective $T_H2$ immune response - the specific $T_H2$ immunity so induced will itself actively suppress the disease. Without the antigen, administration of the immunomodulator would have to be done chronically to continually suppress the inflammation, and the disease would be expected to reappear once treatment was stopped. The long-term effects of continued administration of an anti-inflammatory agent alone might be undesirable. Therefore, it is advantageous to induce active and specific regulation of the inflammatory process by combining an immunomodulator of the invention with a specific antigen.

The invention will now be illustrated by the following non-limiting examples.

## EXAMPLES

### Example 1. Anti-inflammatory effect of oleyl alcohol and other agents-protection against adjuvant arthritis (AA)

One of the models used to test the anti-inflammatory activity of the agents according to the invention is adjuvant arthritis, an experimental disease of the joints inducible in some strains of rats by immunizing them to antigens of *Mycobacterium tuberculosis* (Pearson, 1956). The disease serves as a model of human arthritic conditions such as rheumatoid arthritis, reactive arthritis in Reiter's syndrome, ankylosing spondylitis and other inflammations of the joints which appear to be mediated by the immune system (Pearson, 1964). Adjuvant arthritis also serves as a model of immune-mediated inflammation in general including cell-mediated autoimmune reactions, graft rejection and allergic reaction. For example, treatments which can suppress adjuvant arthritis include immunosuppressive agents such as corticosteroids, cyclosporin A (Jaffee et al., 1989; Pollock et al., 1989), azathioprine, and other immunosuppressive agents which are broadly used in the treatment of autoimmune diseases. Therefore, suppression of adjuvant arthritis by a therapeutic agent indicates that the agent is potentially useful as a broad anti-inflammatory agent.

AA was induced by immunizing inbred 8-10-week old Lewis rats (Harlan-Olac Limited, Blackthorn, Oxon, UK), at the base of the tail with 1 mg/0.1 ml of killed *Mycobacterium tuberculosis* (Sigma) in IFA (Sigma) as described (Pearson, 1956). Arthritis of the limbs was noted to develop 12-14 days later and was scored on a scale of 0-16 summing the severity of the inflammation of each of the 4 limbs on a scale of 0-4, as described (Holoshitz et al., 1983). The peak of the arthritis usually was observed around day 26 after immunization.

Control rats were untreated or treated by injections of saline. A positive control of immunosuppression was obtained by including a group of rats treated with the corticosteroid agent dexamethasone (200 $\mu$g) administered intra-peritoneally every other day beginning on day 12 after induction. The immunomodulator was administered subcutaneously once 14 days before induction of AA or on day 12 after induction of AA. The percent inhibition of inflammation measured on the day of maximal inflammation was computed as follows:

$$\frac{\text{mean maximal score of test group}}{\text{mean maximal score of control group}} \quad \text{X } 100\%$$

Experiments with 100 $\mu$l each of glycerol monooleate, oleyl alcohol, linolenyl alcohol, cetyl alcohol, mannose monooleate, ethyl oleate and methyl oleate showed that all of them were found to be effective, producing more than 66% inhibition of inflammation whereas oleic acid had no effect. The results are summarized in Table 1. Two further experiments showed that 500 $\mu$l of oleyl alcohol suppressed the inflammation by 96% and 91 %.

### Table 1. Effects of various agents on the inflammation of adjuvant arthritis

| Compound Tested | % Inhibition (100 $\mu$l) |
|---|---|
| Glycerol mono-oleate | 98% |
| Oleyl alcohol | 78%-96% |
| Linolenyl alcohol | 75% |
| Cetyl alcohol | 66% |
| Mannose monooleate | 95% |
| Ethyl oleate | 92% |
| Methyl oleate | 76% |

### Example 2. Protection against AA by different doses of oleyl alcohol

To study the dose response effect of oleyl alcohol in AA, oleyl alcohol was administered subcutaneously in doses of 10, 50, 100 or 500 μl to Lewis rats once 14 days before induction of AA as described in Example 1 above.

Fig. 1 shows the dose response effect of oleyl alcohol. It can be seen that increasing doses of oleyl alcohol suppressed the arthritis. On the day of peak disease, day 26, the inflammation was suppressed by 14% (10 μl), 61% (50 μl), 78% (100 μl) and 90% (500 μl).

**Example 3. Use of an immunomodulator as an adjuvant in the treatment of EAE**

Having shown that oleyl alcohol and the other compounds tested in Example 1 above are potent anti-inflammatory immunomodulators, it is of interest now to test their effect as adjuvants together with an antigen recognized by inflammatory T cells associated with the pathogenesis of a T-cell mediated disease such as an autoimmune disease, for the treatment of said T-cell mediated disease.

EAE can be induced in Lewis rats by immunization with MBP in CFA. For this purpose, 7-9 weeks old female Lewis rats are immunized with guinea pig MBP (Sigma) by injecting each foot pad with 25 μg of MBP (50 μg total) in CFA (Sigma). The disease develops about 12 days after immunization and is characterized by paralysis of various degrees due to inflammation of the central nervous system. The rats are scored for paralysis on a four-grade scale: 0, no paralysis; 1, tail weakness (hanging); 2, hind limb paralysis; 3, hind and fore limb paralysis; 4, severe total paralysis (Lorentzen et al., 1995).

EAE is caused by T cells that recognize defined determinants of the MBP molecule. The major MBP determinant in the Lewis rat is composed of the peptide consisting of the sequence 71-90 of MBP (hereinafter p71-90 peptide).

In order to test whether administration of the encephalitogenic MBP p71-90 peptide with an adjuvant of the invention can also inhibit the development of EAE, Lewis rats, groups of 5-8, are treated with subcutaneous injections of the p71-90 peptide emulsified in 100 μl oleyl alcohol or another immunomodulator of the invention in paraffin oil, or with the immunomodulator in paraffin oil alone (without the peptide), on the day of MBP immunization (day 0) and five days later (day 5), or on day - 14 and day - 7 before immunization with MBP. The effect of the treatment with p71-90/oleyl alcohol or another immunomodulator on the EAE is assessed by measuring incidence and severity of the disease. A decrease in the maximal degree of paralysis compared to the control treatment with the p71-90 peptide in paraffin oil or with immunomodulator and paraffin oil without the peptide indicates that a relevant peptide such as p71-90 MBP peptide in an emulsion with said immunomodulator is capable of modulating EAE in rats.

**Example 4. Use of an immunomodulator as an adjuvant in the treatment of AA**

AA is induced in Lewis rats, 7-9 week old females, by immunization at the base of the tail with IFA containing 1 mg of killed Mycobacteria tuberculosis (Sigma; 0.1 ml of 10 mg/ml) as described in Example 1 above. The degree of joint inflammation is graded on a scale of 0-16.

For the treatment of AA, groups of 5-8 females Lewis rats aged 7-9 weeks, after induction of AA, are treated with 100 μg of recombinant hsp60, the mycobacterial epitope recognized by T lymphocytes in Lewis rats adjuvant arthritis (Van Eden et al., 1988) in PBS or emulsified in 100 μl oleyl alcohol or another immunomodulator of the invention in paraffin oil, or with the immunomodulator in paraffin oil alone (without the peptide), by subcutaneous injection on the day of AA induction (day 0) and 7 days later (day + 7), or on day -14 and -7. The effects of the treatment on joint inflammation are assayed as described in Example 1.

**Example 5. Use of an immunomodulator in the treatment of type I diabetes**

In the diabetes NOD mice model, autoimmune destruction of the insulin-producing β-cells in the pancreas is mediated by T-lymphocytes. An inflammatory infiltrate develops around the pancreatic islets at 5-8 weeks of age and β-cell destruction leading to insulin deficiency and overt diabetes becomes manifested at 14-20 weeks of age affecting almost 100% of female NOD mice by 35-40 weeks of age.

Treatment can be performed with the 60 kDa hsp peptide p277 or its analogue p277(Val[6]-Val[11]) (described in WO 97/20016) in a preparation with oleyl alcohol or another immunomodulator of the invention, but is described below for p277(Val[6]-Val[11]) in oleyl alcohol.

NOD female mice are treated with 100 μg p277(Val[6]-Val[11]) in 0.1 ml of PBS or in lipid emulsion of oleyl alcohol. Incidence of diabetes at 6 months of age and the production of anti-p277(Val[6]-Val[11]) antibodies are followed. Diabetes is diagnosed as persistent hyperglycemia, blood glucose levels over 11 mmol/L measured at least twice at weekly intervals with a Beckman Glucose Analyzer II. Successful peptide treatment is assayed by maintenance of a normal blood glucose concentration (less than 11 mmol/L), remission of the intra-islet inflammation of the pancreatic islets (insulitis) and induction of antibodies to the therapeutic peptide as an indicator of a TH2-type immune response.

The protection from diabetes by treatment with the p277(Val[6]-Val[11]) peptide is dependent on TH2 immunological reactivity to the peptide. Therefore, antibody production is measured in the p277(Val[6]-Val[11])-immunized mice by ELISA.

Maxisorp microtiter plates (Nunc) are coated with p277(Val$^6$-Val$^{11}$) peptide, 10 ug/ml, for 18h and non-specific binding blocked with 7% milk powder for 2h. The mouse sera, diluted 1:50, are allowed to bind for 2h and the specific binding is detected by adding alkaline phosphatase anti-mouse IgG (Serotec) for 2h and p-nitrophenylphosphate substrate (Sigma) for 30 min. The color intensity is measured by an ELISA reader (Anthos) at OD=405 nm. NOD mice immunized to p277(Val$^6$-Val$^{11}$) in PBS does not show antibody responses at all, while mice immunized to p277(Val$^6$-Val$^{11}$) in oleyl alcohol will develop peptide specific antibodies, suggesting that the therapeutic effect might result from a shift in the predominant cytokines produced by the autoimmune T cells.

As described before, TH1 cells secrete IL-2, which induces T-cell proliferation, and cytokines such as IFN-γ, which mediates tissue inflammation, while TH2 cells secrete IL-4, which "helps" B cells produce certain antibody isotypes, primarily IgG1 and IgG2b, and IL-10 and other cytokines such as IL-5, which can "depress" tissue inflammation.

The possibility of a shift from TH1 to TH2-type response is verified by analysis of the isotypes of the antibodies produced after p277(Val$^6$-Val$^{11}$) therapy. Groups of NOD mice, 3 months old, are treated with p277(Val$^6$-Val$^{11}$) or with PBS in oil as described above. The sera of individual mice are assayed for the isotypes of their antibodies to p277 (Val$^6$-Val$^{11}$) after treatment (12-15 mice per group). The antibody isotypes are detected using an ELISA assay with isotype-specific developing antibody reagents (Southern Biotechnology Associates, Birmingham, AL). Analysis of the antibody isotypes of the anti-p277 antibodies developed after treatment showing them to be exclusively of the IgG1 and IgG2b classes, dependent on TH2 T cells producing IL-4, confirms that the treatment with the immunomodulator induced a TH1 to TH2 shift. No TH1-type IgG2a antibodies should be produced. The development of antibodies to the specific peptide used in treatment is a sign that the autoimmune T-cell responses have shifted from a damaging TH1 inflammatory mode to a TH2 T-cell response that produces innocuous antibodies and suppresses inflammation and tissue damage.

To confirm the occurrence of a cytokine switch, the cytokines produced by the T-cells reactive to the p277 (Val$^6$-Val$^{11}$) are assayed in the p277(Val$^6$-Val$^{11}$)-oleyl alcohol-treated and control mice. Concanavalin A (ConA), a T-cell mitogen, is used to activate total splenic T-cells as a control.

Groups of 10 NOD mice, 3 months old, are treated with p277(Val$^6$-Val$^{11}$) in oleyl alcohol or with PBS in oleyl alcohol. Five weeks later, the spleens of the mice are removed and the spleen cells are pooled. The spleen cells are incubated with Con A or p277(Val$^6$-Val$^{11}$) for 24h (for IL-2 and IL-4 secretion) or for 48h (for IL-10 and IFN-γ secretion). The presence of the cytokines in the culture supernatants is quantitated by ELISA, using Pharmingen paired antibodies according to the Pharmingen cytokine ELISA protocol. Pharmingen recombinant mouse cytokines are used as standards for calibration curves. Briefly, flat-bottom 96-well microtiter plates are coated with rat anti-mouse cytokine monoclonal antibodies (mAbs) for 18h at 4°C, and the culture supernatants or recombinant mouse cytokines are added for 18h at 4°C. The plates are washed, and biotinylated rat anti-mouse cytokine mAbs are added for 45 min at room temperature, then extensively washed, and avidin-alkaline phosphatase is added. The plates are washed, a chromogen substrate (p-nitrophenylphosphate) is added and samples are read at 405 nm in an ELISA reader. Spleen cells of the control mice secrete both IL-2 and IFN-γ upon incubation with p277(Val$^6$-Val$^{11}$), but produce very little IL-4 or IL-10 in response to p277(Val$^6$-Val$^{11}$) or Con A. A significant increase in IL-10 and IL-4 production in response to p277(Val$^6$-Val$^{11}$) in the p277(Val$^6$-Val$^{11}$)-oleyl alcohol-treated mice coupled with the decrease in IL-2 and IFN-γ production confirms the shift from TH1 to TH2-like behavior.

## REFERENCES

Banchereau, J. and Rybak, M.E. (1994) in: The Cytokine Handbook, 2nd Ed., A, Thompson Ed. Academic Press New York, pp 99.

Ben-Nun, A. and Cohen, I.R. (1982) "Spontaneous remission and acquired resistance to autoimmune encephalomyelitis (EAE) are associated with suppression of T cell reactivity: Suppressed EAE effector T cells recovered as T cell lines. J. Immunol. 128: 1450-1457.

Holoshitz, Y. et al., (1983) "Lines of T lymphocytes mediate or vaccinate against autoimmune arthritis", Science 219: 56.

Jaffee, B.D. et al., (1989) "The effect of immunomodulating drugs on adjuvant-induced arthritis in Lewis rats", Agents Actions. 27 (3-4): 344-6.

Liblau, R.S., Singer, S.M. and McDevitt, H.O. (1995) Immunology Today 16:34-38.

Lorentzen J.C. et al., (1995), "Protracted, relapsing and demyelinating experimental autoimmune encephalomyelitis in DA rats immunized with syngeneic spinal cord and incomplete Freund's adjuvant", J. Neuroimmunology 63: 193-205.

Moore, K.V., O'Garra, A., de Waal Malefyt, R., Vieira, P. and Mosmann, T.R. (1993) Ann. Rev. Immunol. 11:165-190.

Mosmann, T.R. and Coffman R.L. (1989) Ann. Rev. Immunol. 7:145-173

Pearson, CM. (1956) "Development of arthritis, periarthritis and periostitis in rats given adjuvant", Proc. Soc. Exp.

Biol. Med. 91:91.

Pearson, CM. (1964) "Experimental models in rheumatoid disease", Arthritis Rheum. 7:80.

Pollock et al., (1989) "Pharmacokinetic analysis of cyclosporine in adjuvant arthritic rats", Drug Metab. Dispos. 17 (6): 595-9.

Romagnani, S., Ann. Rev. Immunol. (1994) 12:227-257.

Van Eden W, Thole JER, Van Der Zee R, Noordzij A, Embden JDA, Hensen EJ, Cohen IR. (1988) Cloning of the mycobacterial epitope recognized by T lymphocytes in adjuvant arthritis. Nature. 331:171-173.

**Claims**

1. A therapeutic preparation consisting of an antigen and a carrier, wherein said antigen is an antigen recognized by inflammatory T cells associated with the pathogenesis of a T-cell mediated disease or condition, and wherein said carrier is an anti-inflammatory immunomodulator selected from: (a) a saturated or cis-unsaturated $C_{10}$-$C_{20}$ fatty alcohol or an ester thereof with a $C_1$-$C_6$ alkanoic acid; and (b) a saturated or cis-unsaturated $C_{10}$-$C_{20}$ fatty acid ester selected from a $C_1$-$C_6$ alkyl ester, a monoester with a $C_2$-$C_6$ polyol having a least two hydroxy groups, and a diester with glycerol.

2. The therapeutic preparation according to claim 1, wherein the carrier is a saturated $C_{10}$-$C_{20}$ fatty alcohol such as decyl alcohol, lauryl alcohol, myristyl alcohol, cetyl alcohol and stearyl alcohol.

3. The therapeutic preparation according to claim 1, wherein the carrier is a cis-unsaturated $C_{16}$-$C_{18}$ fatty alcohol such as oleyl alcohol, linoleyl alcohol, γ-linolenyl alcohol and linolenyl alcohol.

4. The therapeutic preparation according to claim 1, wherein the carrier is an ester of a saturated or cis-unsaturated $C_{10}$-$C_{20}$ fatty alcohol with a $C_1$-$C_6$ alkanoic acid or a saturated or cis-unsaturated $C_{10}$-$C_{20}$ fatty acid ester selected from a $C_1$-$C_6$ alkyl ester, a monoester with a polyol having at least two hydroxy groups, and a diester with glycerol.

5. The therapeutic preparation according to claim 4, wherein said fatty acid is a saturated $C_{10}$-$C_{20}$ fatty acid such as capric acid, lauric acid, myristic acid, palmitic acid, stearic acid and arachidic acid.

6. The therapeutic preparation according to claim 4, wherein said fatty acid is a cis-unsaturated $C_{10}$-$C_{20}$ fatty acid such as palmitoleic acid, oleic acid, cis-vaccenic acid, linoleic acid, γ -linolenic acid, linolenic acid, and arachidonic acid.

7. The therapeutic preparation according to claim 6, wherein said fatty acid alkyl ester is methyl oleate or ethyl oleate.

8. The therapeutic preparation according to any one of claims 4 to 7, wherein said fatty acid ester is a monoester with a polyol selected from a $C_2$-$C_8$ alkanediol, glycerol and a saccharide.

9. The therapeutic preparation according to claim 8, wherein said alkanediol is selected from 1,2-ethylene glycol, 1,3-propanediol and 1,4-butanediol.

10. The therapeutic preparation according to claim 8, wherein said fatty acid monoester with glycerol is glyceryl monooleate.

11. The therapeutic preparation according to claim 8, wherein said saccharide is a monosaccharide selected from ribose, fructose, glucose, galactose and mannose.

12. The therapeutic preparation according to claim 11, wherein said fatty acid monoester is mannose monooleate.

13. The therapeutic preparation according to any one of claims 4 to 7, wherein said fatty acid ester is a diester with glycerol.

14. The therapeutic preparation according to claim 13, wherein said diglyceride is glyceryl dioleate.

15. Use of a therapeutic preparation according to any one of claims 1-14 for the preparation of a medicament which causes shifting of an individual's T-cell cytokine response from $T_H1$ to $T_H2$.

16. Use according to claim 15 which causes a decrease in IL-2 or IFN-γ T-cell cytokine response and an increase in IL-4 or IL-10 T-cell cytokine response.

17. Use of a therapeutic preparation according to any one of claims 1-14 for the preparation of a medicament for the treatment of a T-cell mediated disease or condition.

18. Use of a therapeutic preparation as defined in any one of claims 1 to 14, wherein said T-cell mediated disease is an autoimmune disease and said antigen is a peptide.

19. Use according to claim 18, wherein said autoimmune disease is an organ-specific autoimmune disease.

20. Use according to claim 19, wherein said organ-specific autoimmune disease is selected from type I diabetes, multiple sclerosis, rheumatoid arthritis and autoimmune thyroiditis.

21. Use according to claim 20, wherein the disease is multiple sclerosis and the peptide is derived from the sequence of myelin basic protein (MBP) or an analogue thereof that is recognized by T-cells involved in the pathogenesis of multiple sclerosis.

22. Use of (i) a carrier which is an anti-inflammatory immunomodulator selected from: (a) a saturated or cis-unsaturated $C_{10}$-$C_{20}$ fatty alcohol or an ester thereof with a $C_1$-$C_6$ alkanoic acid; or (b) a saturated or cis-unsaturated $C_{10}$-$C_{20}$ fatty acid ester selected from a $C_1$-$C_6$ alkyl ester, a monoester with a $C_2$-$C_6$ polyol having at least two hydroxy groups, or a diester with glycerol; and (ii) an antigen recognized by inflammatory T cells associated with the pathogenesis of a T-cell mediated disease or condition, for the manufacture of a therapeutic preparation for the treatment of said T-cell mediated disease or condition.

23. Use according to claim 22, wherein said carrier is as defined in any one of claims 2 to 14.

**Patentansprüche**

1. Therapeutisches Präparat, das aus einem Antigen und einem Träger besteht, wobei das Antigen ein Antigen ist, das von inflammatorischen T-Zellen erkannt wird, die mit der Pathogenese einer T-Zell-vermittelten Erkrankung oder eines T-Zell-vermittelten Zustands in Verbindung gebracht werden, und wobei der Träger ein antiinflammatorischer Immunmodulator ist, der aus (a) einem gesättigten oder cis-ungesättigten $C_{10}$-$C_{20}$-Fettalkohol oder einem Ester davon mit einer $C_1$-$C_6$-Alkansäure; und (b) einem gesättigten oder cis-ungesättigten $C_{10}$-$C_{20}$-Fettsäureester, ausgewählt aus einem $C_1$-$C_6$-Alkylester, einem Monoester mit einem $C_2$-$C_6$-Polyol mit mindestens zwei Hydroxygruppen und einem Diester mit Glycerin, ausgewählt ist.

2. Therapeutisches Präparat nach Anspruch 1, wobei der Träger ein gesättigter $C_{10}$-$C_{20}$-Fettalkohol wie Decylalkohol, Laurylalkohol, Myristylalkohol, Cetylalkohol und Stearylalkohol ist.

3. Therapeutisches Präparat nach Anspruch 1, wobei der Träger ein cis-ungesättigter $C_{16}$-$C_{18}$-Fettalkohol wie Oleylalkohol, Linoleylalkohol, $\gamma$-Linolenylalkohol und Linolenylalkohol ist.

4. Therapeutisches Präparat nach Anspruch 1, wobei der Träger ein Ester eines gesättigten oder cis-ungesättigten $C_{10}$-$C_{20}$-Fettalkohols mit einer $C_1$-$C_6$-Alkansäure oder ein gesättigter oder cis-ungesättigter $C_{10}$-$C_{20}$-Fettsäureester, ausgewählt aus einem $C_1$-$C_6$-Alkylester, einem Monoester mit einem Polyol mit mindestens zwei Hydroxygruppen und einem Diester mit Glycerin, ist.

5. Therapeutisches Präparat nach Anspruch 4, wobei die Fettsäure eine gesättigte $C_{10}$-$C_{20}$-Fettsäure wie Caprinsäure, Laurinsäure, Myristinsäure, Palmitinsäure, Stearinsäure und Arachinsäure ist.

6. Therapeutisches Präparat nach Anspruch 4, wobei die Fettsäure eine cis-ungesättigte $C_{10}$-$C_{20}$-Fettsäure wie Palmitoleinsäure, Ölsäure, cis-Vaccensäure, Linolsäure, $\gamma$-Linolensäure, Linolensäure und Arachidonsäure ist.

7. Therapeutisches Präparat nach Anspruch 6, wobei der Fettsäurealkylester Methyloleat oder Ethyloleat ist.

8. Therapeutisches Präparat nach einem der Ansprüche 4 bis 7, wobei der Fettsäureester ein Monoester mit einem Polyol, ausgewählt aus einem $C_2$-$C_8$-Alkandiol, Glycerin und einem Saccharid, ist.

9. Therapeutisches Präparat nach Anspruch 8, wobei das Alkandiol aus 1,2-Ethylenglykol, 1,3-Propandiol und 1,4-Butandiol ausgewählt ist.

10. Therapeutisches Präparat nach Anspruch 8, wobei der Fettsäuremonoester mit Glycerin Glycerylmonooleat ist.

11. Therapeutisches Präparat nach Anspruch 8, wobei das Saccharid ein Monosaccharid, ausgewählt aus Ribose, Fructose, Glucose, Galactose und Mannose, ist.

12. Therapeutisches Präparat nach Anspruch 11, wobei der Fettsäuremonoester Mannosemonooleat ist.

13. Therapeutisches Präparat nach einem der Ansprüche 4 bis 7, wobei der Fettsäureester ein Diester mit Glycerin ist.

14. Therapeutisches Präparat nach Anspruch 13, wobei das Diglycerid Glyceryldioleat ist.

15. Verwendung eines therapeutischen Präparats nach einem der Ansprüche 1 bis 14 für die Herstellung eines Medikaments, das bei einem Patienten eine Verschiebung der T-Zell-Cytokin-Antwort von $T_H1$ in Richtung $T_H2$ bewirkt.

16. Verwendung nach Anspruch 15, welche eine Abnahme der IL-2- oder IFN-$\gamma$-T-Zell-Cytokin-Antwort und eine Zunahme der IL-4- oder IL-10-T-Zell-Cytokin-Antwort bewirkt.

17. Verwendung eines therapeutischen Präparats nach einem der Ansprüche 1 bis 14 für die Herstellung eines Medikaments zur Behandlung einer T-Zell-vermittelten Erkrankung oder eines T-Zell-vermittelten Zustands.

18. Verwendung eines therapeutischen Präparats, wie in einem der Ansprüche 1 bis 14 definiert, wobei die T-Zell-vermittelte Erkrankung eine Autoimmunerkrankung ist und das Antigen ein Peptid ist.

19. Verwendung nach Anspruch 18, wobei die Autoimmunerkrankung eine organspezifische Autoimmunerkrankung ist.

20. Verwendung nach Anspruch 19, wobei die organspezifische Autoimmunerkrankung aus Typ-1-Diabetes, Multipler Sklerose, rheumatoider Arthritis und Autoimmunthyroiditis ausgewählt ist.

21. Verwendung nach Anspruch 20, wobei die Erkrankung Multiple Sklerose ist und das Peptid aus der Sequenz des Myelin-Basischen Proteins (MBP) oder eines Analogons davon stammt, welches von T-Zellen erkannt wird, die an der Pathogenese von Multipler Sklerose beteiligt sind.

22. Verwendung (i) eines Trägers, bei dem es sich um einen antiinflammatorischen Immunmodulator handelt, ausgewählt aus (a) einem gesättigten oder cis-ungesättigten $C_{10}$-$C_{20}$-Fettalkohol oder einem Ester davon mit einer $C_1$-$C_6$-Alkansäure; oder (b) einem gesättigten oder cis-ungesättigten $C_{10}$-$C_{20}$-Fettsäureester, ausgewählt aus einem $C_1$-$C_6$-Alkylester, einem Monoester mit einem $C_2$-$C_6$-Polyol mit mindestens zwei Hydroxygruppen oder einem Diester mit Glycerin, und (ii) eines Antigens, das von inflammatorischen T-Zellen erkannt wird, die mit der Pathogenese einer T-Zell-vermittelten Erkrankung oder eines T-Zell-vermittelten Zustands in Verbindung gebracht werden, für die Herstellung eines therapeutischen Präparats zur Behandlung der T-Zell-vermittelten Erkrankung oder des T-Zell-vermittelten Zustands.

23. Verwendung nach Anspruch 22, wobei der Träger wie in einem der Ansprüche 2 bis 14 definiert ist.

**Revendications**

1. Préparation thérapeutique consistant en un antigène et un transporteur, dans laquelle ledit antigène est un antigène reconnu par des lymphocytes T inflammatoires associés à la pathogenèse d'une maladie ou d'un état induit(e) par les lymphocytes T, et dans laquelle ledit transporteur est un immunomodulateur anti-inflammatoire choisi parmi : (a) un alcool gras en $C_{10}$ à $C_{20}$ saturé ou cis-insaturé ou un ester de celui-ci avec un acide alcanoïque en $C_1$ à $C_6$ ; et (b) un ester d'acide gras en $C_{10}$ à $C_{20}$ saturé ou cis-insaturé choisi parmi un ester d'alkyle en $C_1$ à $C_6$, un monoester avec un polyol en $C_2$ à $C_6$ ayant au moins deux groupes hydroxy, et un diester avec du glycérol.

2. Préparation thérapeutique selon la revendication 1, dans laquelle le transporteur est un alcool gras en $C_{10}$ à $C_{20}$ saturé tel que l'alcool décylique, l'alcool laurique, l'alcool myristique, l'alcool cétylique et l'alcool stéarique.

**3.** Préparation thérapeutique selon la revendication 1, dans laquelle le transporteur est un alcool gras en $C_{16}$ à $C_{18}$ cis-insaturé tel que l'alcool oléique, l'alcool linoléique, l'alcool $\gamma$-linolénique et l'alcool linolénique.

**4.** Préparation thérapeutique selon la revendication 1, dans laquelle le transporteur est un ester d'un alcool gras en $C_{10}$ à $C_{20}$ saturé ou cis-insaturé avec un acide alcanoïque en $C_1$ à $C_6$ ou un ester d'acide gras en $C_{10}$ à $C_{20}$ saturé ou cis-insaturé choisi parmi un ester d'alkyle en $C_1$ à $C_6$, un monoester avec un polyol ayant au moins deux groupes hydroxy, et un diester avec du glycérol.

**5.** Préparation thérapeutique selon la revendication 4, dans laquelle ledit acide gras est un acide gras en $C_{10}$ à $C_{20}$ saturé tel que l'acide caprique, l'acide laurique, l'acide myristique, l'acide palmitique, l'acide stéarique et l'acide arachidique.

**6.** Préparation thérapeutique selon la revendication 4, dans laquelle ledit acide gras est un acide gras en $C_{10}$ à $C_{20}$ cis-insaturé tel que l'acide palmitoléique, l'acide oléique, l'acide cis-vaccénique, l'acide linoléique, l'acide $\gamma$-linolénique, l'acide linolénique, et l'acide arachidonique.

**7.** Préparation thérapeutique selon la revendication 6, dans laquelle ledit ester d'alkyle d'acide gras est l'oléate de méthyle ou l'oléate d'éthyle.

**8.** Préparation thérapeutique selon l'une quelconque des revendications 4 à 7, dans laquelle ledit ester d'acide gras est un monoester avec un polyol choisi parmi un alcanediol en $C_2$ à $C_8$, du glycérol et un saccharide.

**9.** Préparation thérapeutique selon la revendication 8, dans laquelle ledit alcanediol est choisi parmi le 1,2-éthylène glycol, le 1,3-propanediol et le 1,4-butanediol.

**10.** Préparation thérapeutique selon la revendication 8, dans laquelle ledit monoester d'acide gras avec du glycérol est le monooléate de glycéryle.

**11.** Préparation thérapeutique selon la revendication 8, dans laquelle ledit saccharide est un monosaccharide choisi parmi le ribose, le fructose, le glucose, le galactose et le mannose.

**12.** Préparation thérapeutique selon la revendication 11, dans laquelle ledit monoester d'acide gras est le monooléate de mannose.

**13.** Préparation thérapeutique selon l'une quelconque des revendications 4 à 7, dans laquelle ledit ester d'acide gras est un diester avec du glycérol.

**14.** Préparation thérapeutique selon la revendication 13, dans laquelle ledit diglycéride est le dioléate de glycéryle.

**15.** Utilisation d'une préparation thérapeutique selon l'une quelconque des revendications 1 à 14 pour la préparation d'un médicament qui entraîne une variation d'une réponse cytokinique des lymphocytes T chez un individu de $T_H1$ à TH$_2$.

**16.** Utilisation selon la revendication 15 qui entraîne une diminution de la réponse cytokinique des lymphocytes T en IL-2 ou en IFN-$\gamma$ et une augmentation de la réponse cytokinique des lymphocytes T en IL-4 ou en IL-10.

**17.** Utilisation d'une préparation thérapeutique selon l'une quelconque des revendications 1 à 14 pour la préparation d'un médicament pour le traitement d'une maladie ou d'un état induit(e) par les lymphocytes T.

**18.** Utilisation d'une préparation thérapeutique telle que définie dans l'une quelconque des revendications 1 à 14, dans laquelle ladite maladie induite par les lymphocytes T est une maladie auto-immune et ledit antigène est un peptide.

**19.** Utilisation selon la revendication 18, dans laquelle ladite maladie auto-immune est une maladie auto-immune spécifique d'un organe.

**20.** Utilisation selon la revendication 19, dans laquelle ladite maladie auto-immune spécifique d'un organe est choisie parmi les diabètes de type I, la sclérose en plaques, l'arthrite rhumatoïde et la thyroïdite auto-immune.

**21.** Utilisation selon la revendication 20, dans laquelle la maladie est la sclérose en plaques et le peptide est dérivé de la séquence de la protéine basique de myéline (MBP) ou d'un analogue de celle-ci qui est reconnu(e) par les lymphocytes T dans la pathogenèse de la sclérose en plaques.

**22.** Utilisation de (i) un transporteur qui est un immunomodulateur anti-inflammatoire choisi parmi : (a) un alcool gras en $C_{10}$ à $C_{20}$ saturé ou cis-insaturé ou un ester de celui-ci avec un acide alcanoïque en $C_1$ à $C_6$ ; ou (b) un ester d'acide gras en $C_{10}$ à $C_{20}$ saturé ou cis-insaturé choisi parmi un ester d'alkyle en $C_1$ à $C_6$, un monoester avec un polyol en $C_2$ à $C_6$ ayant au moins deux groupes hydroxy, ou un diester avec du glycérol ; et (ii) un antigène reconnu par des lymphocytes T inflammatoires associés à la pathogenèse d'une maladie ou d'un état induit(e) par les lymphocytes T, pour la fabrication d'une préparation thérapeutique pour le traitement de ladite maladie ou de ledit état induit(e) par les lymphocytes T.

**23.** Utilisation selon la revendication 22, dans laquelle ledit transporteur est tel que défini dans l'une quelconque des revendications 2 à 14.

Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5817629 A
- US 6250040 B
- US 5948764 A
- US 6329499 B
- US 5945401 A
- US 6197926 B
- US 5961977 A

- WO 97020016 A
- US 5019383 A
- US 5984764 A
- WO 9904632 A
- US 6239499 A
- US 6066621 A
- WO 9720016 A

**Non-patent literature cited in the description**

- **Heeger et al.** *J. Immunol.,* 2000, vol. 164, 5771-5781
- **Banchereau, J. ; Rybak, M.E.** The Cytokine Handbook. Academic Press, 1994, 99
- **Ben-Nun, A. ; Cohen, I.R.** Spontaneous remission and acquired resistance to autoimmune encephalomyelitis (EAE) are associated with suppression of T cell reactivity: Suppressed EAE effector T cells recovered as T cell lines. *J. Immunol.,* 1982, vol. 128, 1450-1457
- **Holoshitz, Y. et al.** Lines of T lymphocytes mediate or vaccinate against autoimmune arthritis. *Science,* 1983, vol. 219, 56
- **Jaffee, B.D. et al.** The effect of immunomodulating drugs on adjuvant-induced arthritis in Lewis rats. *Agents Actions,* 1989, vol. 27 (3-4), 344-6
- **Liblau, R.S. ; Singer, S.M. ; McDevitt, H.O.** *Immunology Today,* 1995, vol. 16, 34-38
- **Lorentzen J.C. et al.** Protracted, relapsing and demyelinating experimental autoimmune encephalomyelitis in DA rats immunized with syngeneic spinal cord and incomplete Freund's adjuvant. *J. Neuroimmunology,* 1995, vol. 63, 193-205

- **Moore, K.V. ; O'Garra, A. ; de Waal Malefyt, R. ; Vieira, P. ; Mosmann, T.R.** *Ann. Rev. Immunol.,* 1993, vol. 11, 165-190
- **Mosmann, T.R. ; Coffman R.L.** *Ann. Rev. Immunol.,* 1989, vol. 7, 145-173
- **Pearson, CM.** Development of arthritis, periarthritis and periostitis in rats given adjuvant. *Proc. Soc. Exp. Biol. Med.,* 1956, vol. 91, 91
- **Pearson, CM.** Experimental models in rheumatoid disease. *Arthritis Rheum.,* 1964, vol. 7, 80
- **Pollock et al.** Pharmacokinetic analysis of cyclosporine in adjuvant arthritic rats. *Drug Metab. Dispos.,* 1989, vol. 17 (6), 595-9
- **Romagnani, S.** *Ann. Rev. Immunol.,* 1994, vol. 12, 227-257
- **Van Eden W ; Thole JER ; Van Der Zee R ; Noordzij A ; Embden JDA ; Hensen EJ ; Cohen IR.** Cloning of the mycobacterial epitope recognized by T lymphocytes in adjuvant arthritis. *Nature,* 1988, vol. 331, 171-173